# EUROPEAN PATENT SPECIFICATION

(11) **EP 0 968 230 B1**
(45) Date of publication and mention of the grant of the patent: **11.10.2006**
(21) Application number: 98912581.0
(22) Date of filing: 20.03.1998
(51) Int. Cl.: C07K 14/75, C07K 16/18, G01N 33/68, A61K 38/17

(54) **OXIDIZED FRAGMENTS OF APOLIPOPROTEIN B AND THEIR USE**
OXIDIERTE FRAGMENTE DES APOLIPOPROTEIN B UND IHRE VERWENDUNG
FRAGMENTS OXYDES D'APOLIPOPROTEINE B ET UTILISATIONS

(30) Priority: 20.03.1997 GB 9705831
(43) Date of publication of application: 05.01.2000
(73) Proprietor: UNIVERSITY OF LEICESTER, Leicester, Leicestershire LE1 7RH (GB)
(72) Inventor: LUNEC, Joe, West Midlands B91 1JU (GB); BEVAN, Ruth, Nottingham NG2 6RG (GB); GRIFFITHS, Helen, Solihull, West Midlands B93 8ET (GB)
(74) Representative: Arends, William Gerrit
(86) International application number: PCT/GB1998/000677
(87) International publication number: WO 1998/042751

(56) References cited:
- H.F. HOFF AND J. O'NEILL: "Lesion-Derived Low Density Lipoprotein and Oxidized Low Density Lipoprotein Share a Lability for Aggregation, Leading to Enhanced Macrophage Degradation" ARTHEROSCLEROTIS AND THROMBOSIS, vol. 11, no. 5, September 1991 - October 1991, pages 1209-1222, XP002070326
- S. YLÄ-HERTTUALA ET AL.: "Evidence for the Presence of Oxidatively Modified Low Density Lipoprotein in Atherosclerotic Lesions of Rabbit and Man" JOURNAL OF CLINICAL INVESTIGATION, vol. 84, no. 4, October 1989, pages 1086-1095, XP002070327
- Q. CHEN ET AL.: "Studies on epitopes on low-dnsity lipoprotein modified by 4-hydroynonenal" BIOCHEMICAL JOURNAL , vol. 288, no. 1, 15 November 1992, pages 249-254, XP002070328 cited in the application

## Description

Atherosclerosis is a major cause of mortality in all western populations. High serum cholesterol levels are associated with increased cardiovascular risk (Marmot, M., 1988, Atherosclerosis Reviews, 18: 95-108; WHO Monica Project, 1988, World Health Statistics Quarterly, 41: 115-138). However, oxidised sterols (cholesterol being a sterol) and not cholesterol *per se* appear to be the true agents which induce atherosclerotic lesions. Low levels of antioxidants, particularly vitamin E and B carotene are also associated with an increased risk of cardiovascular disease (Diplock, A.T., 1994, Mol. Asp. Med., 115: 295-376). These data suggest a role for the oxidation of lipids in the aetiopathogenesis of atherosclerosis.

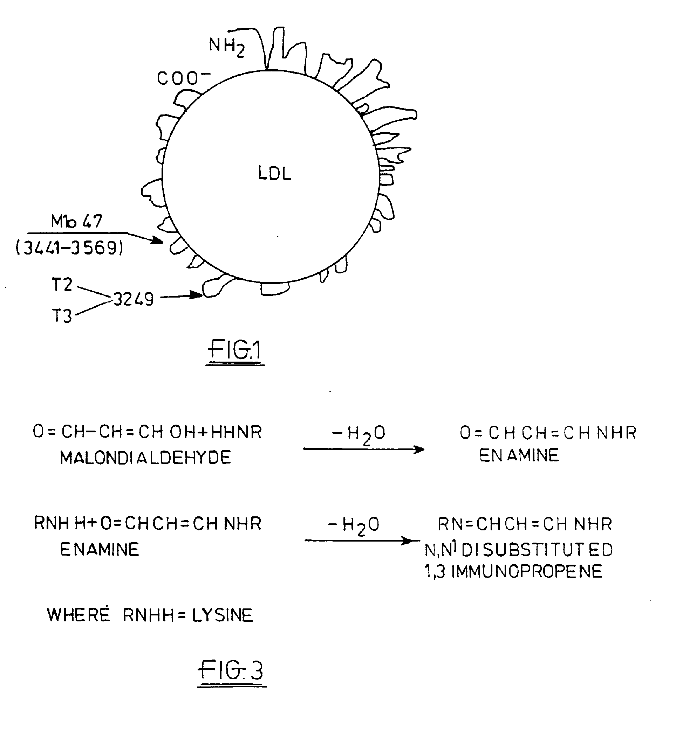

Circulating cholesterol is contained primarily in apoprotein B - based low density lipoprotein (LDL) - a spheroidal particle comprising approximately 1500 cholesterol ester molecules surrounded by a layer of 800 phospholipid molecules, 500 cholesterol molecules and at least one 550 kDa molecule of apoprotein B 100 (Apo B) (Figure 1). Increased levels of LDL correlate strongly with accelerated atherosclerosis. Atherosclerosis is characterised by thickening and degeneration of the arterial intima, the pathogenesis falling into two defined stages - a first stage in which fatty streaks containing foam cells form in the intima, and a second stage in which fibrous plaques are formed within the artery.

Foam cells are formed from macrophages when oxidised LDL is endocytosed by the macrophages *via* a scavenger receptor. This fools the cell into believing that it has taken up too little cholesterol, causing cholesterol to enter the cell *via* the high affinity LDL receptors (which recognise predominantly the Apo B portion of LDL) in an uncontrolled manner (Steinberg, D. *et al.,* 1989, N. Eng. J. Med., 320 (14): 915-924; Goldstein, J.L. *et al.,* 1979, Proc. Natl. Acad. Sci. USA., 76 (1): 333-337) instead of its usual precisely controlled manner. This saturation of the macrophage with cholesterol results in its morphological change into a foam cell.

The oxidation of LDL results in the covalent binding of the oxidation products of the fatty acids on LDL with amino acid residues of the LDL proteins, including tryptophan, arginine, histidine and lysine, and resulting in the neutralisation of the positive charges on the amino acids (Esterbauer, H. *et al.,* 1987, J. Lipid, Res., 28: 495-509; Chen. *Q. et al.,* 1992, Biochem. J., 288: 249-254). Particular modification products of the fatty acids include malondialdehyde (MDA) (formed by the degradation of lipid peroxides) and 4-hydroxynonenal. Modification of LDL can be similarly achieved by glycation of Apo B (for example in poorly controlled diabetics), or by direct oxidation events. Tryptophan can also be modified to give N-formylkynurenine and bityrosine.

The present inventors have now identified peptide fragments of the apoprotein B 100 portion of LDL which are oxidised and which present epitopes which prevent oxidised LDL from being uptaken by the scavenger receptor, thereby preventing the uptake of LDL by the high affinity LDL receptor.

According to the present invention there is provided a molecule consisting of the sequence ALQYKLEGTTR (SEQ ID NO: 1), lysine 5 being conjugated with MDA, and which inhibits uptake by the high affinity LDL receptor of LDL or a partially modified form thereof. The sequence is residues 3349-3359 of apoprotein B 100 (full Apoprotein B sequence - Knolt, T.J. *et al.,* 1986, Nature, 734-738), lysine 5 corresponding to residue 3353.

Also provided according to the present invention is a molecule consisting of the sequence RLTRKRGLKLA (SEQ ID NO: 2), lysine 5 being conjugated with MDA, and which inhibits uptake by the high affinity LDL receptor of LDL or a partially modified form thereof. The sequence is residues 3359-3369 of apoprotein B 100, lysine 5 corresponding to residue 3363

Also provided according to the present invention is a molecule consisting of the sequence ALSLSNKFVEG (SEQ ID NO: 3), lysine 7 being conjugated with MDA, and which inhibits uptake by the high affinity LDL receptor of LDL or a partially modified form thereof. The sequence is residues 3371-3381 of apoprotein B 100, lysine 7 corresponding to residue 3377.

Modification of LDL may for example be by conjugation with MDA, by glycation or by conjugation with hydroxy alkenals such as 4-hydroxynonenal.

Molecules according to the present invention may be for use as immunogens, e.g. for the production of antibodies (or antigen binding fragments thereof) against them (Harlow, E. and Lane, D., "Antibodies - A Laboratory Manual", Cold Spring Harbor Laboratory, Cold Spring Harbor Press, New York, 1988). The term "antibody" is used to describe any antigen-binding species, for example an antigen-binding antibody fragment.

The molecules according to the present invention may be for use in a method of treatment or diagnosis of the human or animal body. Particularly, they may be for the treatment of diagnosis of atherosclerosis. Treatment may of course be both prophylactic and therapeutic.

Also provided according to the present invention is the use of a molecule according to the present invention in the manufacture of a medicament for inhibiting uptake by the high affinity LDL receptor of LDL or a partially modified form thereof. Also provided is a method of manufacture of same medicament, comprising the use of a molecule according to the present invention.

Methods of manufacture are well known in the art. For example, a molecule according to the present invention may be provided with a pharmaceutically acceptable carrier, diluent or excipient (Remington's Pharmaceutical Sciences and US Pharmacopeia, 1984, Mack Publishing Company, Easton, PA, USA), ready for e.g. intravenous injection. Similarly, the dose to give may be readily determined using standard *in vitrolin vivo* does-response experiments. Culture systems for macrophages are well known and may be readily employed in such experiments.

Also provided according to the present invention is the use of antibody which binds specifically with molecules of the present invention in the manufacture of a medicament for inhibiting uptake by the high affinity LDL receptor of LDL or a partially modified form thereof. Also provided is a method of manufacture of same medicament, comprising the use of antibody which binds specifically with molecules of the present invention. A particular use of the antibody is in the treatment or diagnosis of atherosclerosis, treatment being both prophylactic and therapeutic.

Also provided according to the present invention is the use of a molecule according to the present invention in a diagnostic test method for antibody specific against oxidised LDL which will cause uptake by the high affinity LDL receptor of LDL or a partially modified form thereof.

Also provided is a diagnostic test method for antibody specific against oxidised LDL which will cause uptake by the high affinity LDL receptor of LDL or a partially modified form thereof, comprising the steps of:
i) reacting a molecule according to the present invention with a sample;
ii) detecting an antibody-antigen binding reaction; and
iii) correlating detection of the antibody-antigen binding reaction with the presence of antibody specific against oxidised LDL which will cause uptake by the high affinity LDL receptor of LDL or a partially modified form thereof.

Also disclosed herein is a diagnostic test method for modified or conjugated LDL or peptides of the same which will not be taken up by the high affinity of LDL receptor, comprising the steps of:
i) reacting an antibody or antigen binding fragment specific to a molecule according to the present invention with a sample;
ii) detecting an antibody-antigen binding reaction; and
iii) correlating detection of the antibody-antigen binding reaction with the presence of oxidised or conjugated LDL or peptides of the same which will not be taken up by the high affinity LDL receptor.

The sample may be a patient plasma sample.

The plasma may contain antigen representing various types of oxidation and conjugation.

Molecules according to the present invention and antibodies which bind specifically therewith may be used to quantitatively standardise results obtained from such diagnostic tests, or may be used as reagents in the tests. They may have therapeutic benefit as antagonists and could prevent foam cell formation.

Diagnostic test methods may include ELISA (enzyme-linked immunosorbent assay), for example antigen capture ELISA or competitive ELISA, immunoturbidimetry or dip-stick assays (WO 88/08534).

In order to test for specific conditions and causes of oxidation of LDL (for example oxidation caused by hyperglycaemia, hypercholesterolaemia, systemic lupus erythematosis (SLE) or hereditary conditions, molecules according to the present invention modified by for example glycation or conjugation with MDA may be used as appropriate, as may antibody specific to the molecules.

Also provided according to the present invention is a diagnostic test kit for performing a diagnostic test method according to the present invention. Such a kit may include instructions for its use (i.e. for performing an appropriate diagnostic test method according to the present invention).

The invention will be further apparent from the following description, with reference to the several figures of the accompanying drawings, which show, by way of example only, methods of detection of oxidised or conjugated LDL and of peptides of same. Of the figures:
Figure 1 shows a schematic representation of an LDL particle and Apo B epitopes. The location of Apo B fragment responsible for LDL binding to the high affinity LDL receptor is shown as the amino acid residues 3441-3569 defined by monoclonal antibody Mb47 (Knolt, T.J. *et al., supra*). Mb47 blocks LDL binding to the high affinity LDL receptor. T₂T₃ suggests the boundary of thrombolytic peptides;

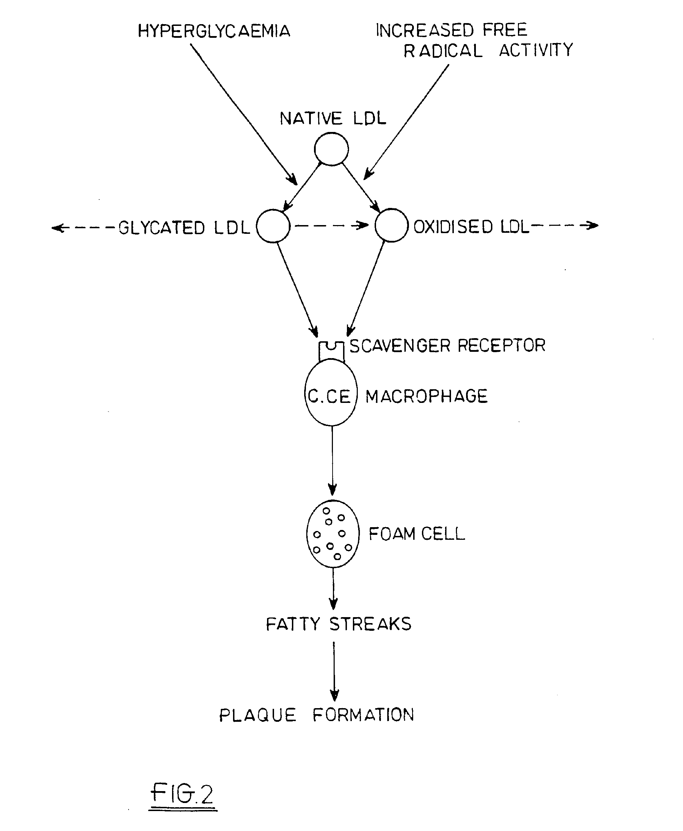

Figure 2 shows the common mechanism of modification of LDL through glycation and conjugation with MDA. Increased free radical activity caused by non-enzymic glycosylation, the polyol pathway, reduced antioxidant reserves and activated neutrophils etc. causes lipid peroxides, lipid peroxy radicals, lipid alkoxyl radicals and aldehydes to convert native LDL to oxidised LDL, causing endothelial damage and smooth muscle cell damage. Glycated LDL results in increased platelet aggregation, increased covalent binding to vascular matrix proteins, and endothelial damage;

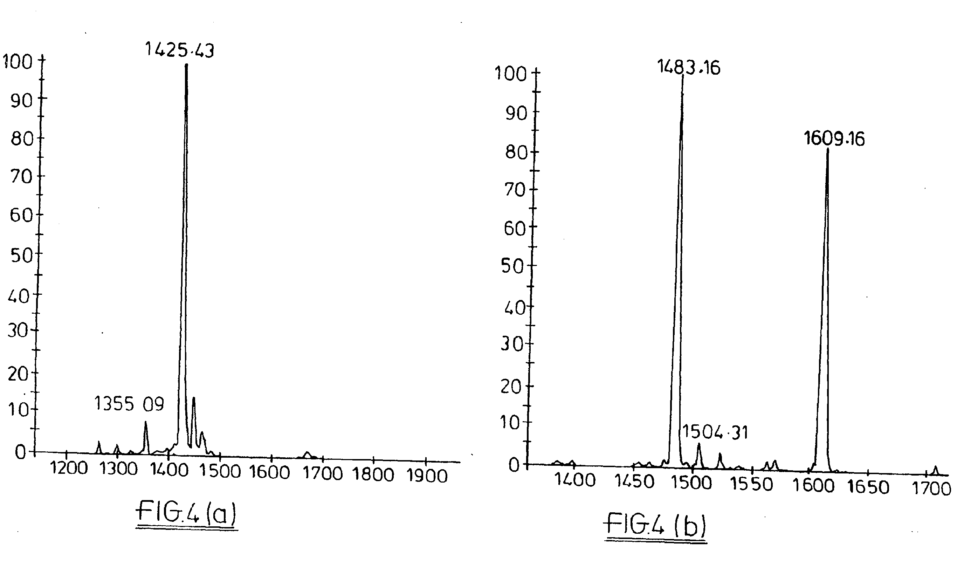

Figure 3 shows the conjugation of MDA with lysine; and
Figure 4 shows a comparison of peptide ALQYKLEGTTR (SEQ ID NO: 1) before (a) and after (b) conjugation with MDA. X axis shows the mass/charge ratio; Y axis shows relative abundance. Numbered peaks are at (Figure 4a) 1355.09 and 1425.43 and (figure 4b) at 1483.16, 1504.31 and 1609.16 on the X-axis.

### Experimental

The peptide sequence ALQYKLEGTTR (SEQ ID NO: 1) was synthesised using standard methods and conjugated with MDA. Figure 4 shows the results of a mass spectrometry plot of the peptide before and after conjugation. Molecular weights of products are consistent with peak 1 (Fig. 4(b)) being conjugated with a single molecule of MDA and peak 2 (Fig. 4(b)) being conjugated to a dimeric form of MDA.

### Antigen capture ELISA

Antibody specific against an antigen is coated onto an ELISA plate and used to capture antigen from patient plasma - either total plasma or LDL fractions are used. Binding is then detected using a second antibody specific against the antigen followed by an enzyme-conjugated anti-immunoglobulin for colorimetric detection.

### Competitive ELISA

An ELISA plate is coated with either MDA-conjugated ALQYKLEGTTR (SEQ ID NO: 1) peptides or with oxidised or conjugated LDL or peptides of the same which will not be taken up by the high affinity LDL receptor. Serial dilutions of patient serum (for example total plasma or LDL fractions can be added) are added together with antibody of fixed dilution. Binding of antibody to coating antigen is detected using enzyme-conjugated anti-immunoglobulin, the extent of binding reducing as the concentration of antigen in the plasma increases. Results are standardised by producing a standard curve using MDA-conjugated ALQYKLEGTTR (SEQ ID NO: 1) peptides.

### Immunoturbidimetry

Latex beads are coated with antibody specific to MDA-conjugated ALQYKLEGTTR (SEQ ID NO: 1) peptides, and the beads mixed with patient sera. The aggregation of the beads due to antibody cross-linking in the presence of specific antigen is analysed in autoanalyzers using immunoturbidimetry detection systems.

The procedures described above are used in diagnosis, for example in ELISA, immunoturbidimetry or dip-stick assays and test kits.

## Claims

1. A molecule consisting of the sequence of SEQ ID NO: 1, lysine 5 being conjugated with MDA, and which inhibits uptake by the high affinity LDL receptor of LDL.

2. A molecule consisting of the sequence of SEQ ID NO: 2, lysine 5 being conjugated with MDA, and which inhibits uptake by the high affinity LDL receptor of LDL.

3. A molecule consisting of the sequence of SEQ ID NO: 3, lysine 7 being conjugated with MDA, and which inhibits uptake by the high affinity LDL receptor of LDL.

4. A molecule according to any of claims 1 to 3, which inhibits uptake of LDL modified by conjugation with MDA.

5. A molecule according to any one of claims 1 to 3, which inhibits uptake of LDL modified by glycation.

6. A molecule according to any one of claims 1 to 3, which inhibits uptake of LDL modified by conjugation with hydroxy alkenals.

7. A molecule according to claim 6, the conjugation being with 4-hydroxynonenal.

8. A molecule according to any of the preceding claims for use in a method of treatment or diagnosis of the human or animal body.

9. The use of a molecule according to any of claims 1-7 in the manufacture of a medicament for inhibiting uptake by the high affinity LDL receptor of LDL.

10. A method of manufacture of a medicament for inhibiting the uptake by the high affinity LDL receptor of LDL, comprising the use of a molecule according to any of claims 1-7.

11. The use of antibody which binds specifically with a molecule according to any of claims 1-7 in the manufacture of a medicament for inhibiting uptake by the high affinity LDL receptor of LDL.

12. A method of manufacture of a medicament for inhibiting uptake by the high affinity LDL receptor of LDL or a partially modified form thereof, comprising the use of antibody which binds specifically with a molecule according to any one of claims 1-7.

13. The use of a molecule according to any of claims 1-7 in a diagnostic test method for antibody specific against oxidised LDL which will cause uptake by the high affinity LDL receptor of LDL or a partially modified form thereof.

14. A diagnostic test method for antibody specific against oxidised LDL which will cause uptake by the high affinity LDL receptor of LDL, comprising the steps of:
i) reacting a molecule according to any of claims 1-7 with a sample;
ii) detecting an antibody-antigen binding reaction; and
iii) correlating detection of the antibody-antigen binding reaction with the presence of antibody specific against oxidised LDL which will cause uptake by the high affinity LDL receptor of LDL or a partially modified form thereof.

15. A diagnostic test method according to claim 14, the sample being a patient plasma sample.

16. A diagnostic test kit for performing a diagnostic test method according to either of claims 14 or 15.

## Patentansprüche

1. Molekül, das aus der Sequenz von SEQ ID NO: 1 besteht, wobei Lysin 5 mit MDA konjugiert ist, und das die Aufnahme von LDL durch den hochaffinen LDL-Rezeptor inhibiert.

2. Molekül, das aus der Sequenz von SEQ ID NO: 2 besteht, wobei Lysin 5 mit MDA konjugiert ist, und das die Aufnahme von LDL durch den hochaffinen LDL-Rezeptor inhibiert.

3. Molekül, das aus der Sequenz von SEQ ID NO: 3 besteht, wobei Lysin 5 mit MDA konjugiert ist, und das die Aufnahme von LDL durch den hochaffinen LDL-Rezeptor inhibiert.

4. Molekül nach einem der Ansprüche 1 bis 3, das die Aufnahme von LDL inhibiert, das durch Konjugation mit MDA modifiziert ist.

5. Molekül nach einem der Ansprüche 1 bis 3, das die Aufnahme von LDL inhibiert, das durch Glykierung modifiziert ist.

6. Molekül nach einem der Ansprüche 1 bis 3, das die Aufnahme von LDL inhibiert, das durch Konjugation mit Hydroxyalkenalen modifiziert ist.

7. Molekül nach Anspruch 6, wobei die Konjugation mit 4-Hydroxynonenal ist.

8. Molekül nach einem der vorhergehenden Ansprüche zur Verwendung bei einem Verfahren zur Behandlung oder Diagnose des menschlichen oder tierischen Körpers.

9. Verwendung eines Moleküls nach einem der Ansprüche 1 - 7 bei der Herstellung eines Arzneimittels zum Inhibieren der Aufnahme von LDL durch den hochaffinen LDL-Rezeptor.

10. Verfahren zur Herstellung eines Arzneimittels zum Inhibieren der Aufnahme von LDL durch den hochaffinen LDL-Rezeptor, das die Verwendung eines Moleküls nach einem der Ansprüche 1 - 7 umfasst.

11. Verwendung von Antikörper, der spezifisch mit einem Molekül nach einem der Ansprüche 1 -7 bindet, bei der Herstellung eines Arzneimittels zum Inhibieren der Aufnahme von LDL durch den hochaffinen LDL-Rezeptor.

12. Verfahren zur Herstellung eines Arzneimittels zum Inhibieren der Aufnahme von LDL oder einer teilweise modifizierten Form davon durch den hochaffinen LDL-Rezeptor, das die Verwendung eines Antikörpers umfasst, der spezifisch mit einem Molekül nach einem der Ansprüche 1 -7 bindet.

13. Verwendung eines Moleküls nach einem der Ansprüche 1 - 7 in einem diagnostischen Testverfahren auf Antikörper, der spezifisch gegen oxidiertes LDL ist, das die Aufnahme von LDL oder einer teilweise modifizierten Form durch den hochaffinen LDL-Rezeptor bewirken wird.

14. Diagnostisches Testverfahren auf Antikörper, der spezifisch gegen oxidiertes LDL ist, das die Aufnahme von LDL durch den hochaffinen LDL-Rezeptor bewirken wird, das die folgenden Schritte umfasst:
i) Reagieren eines Moleküls nach einem der Ansprüche 1 - 7 mit einer Probe;
ii) Nachweisen einer Antikörper-Antigen-Bindungsreaktion und
iii) Korrelieren des Nachweises der Antikörper-Antigen-Bindungsreaktion mit der Gegenwart von Antikörper, der spezifisch gegen oxidiertes LDL ist, das die Aufnahme von LDL oder einer teilweise modifizierten Form durch den hochaffinen LDL-Rezeptor bewirken wird.

15. Diagnostisches Testverfahren nach Anspruch 14, wobei die Probe eine Patientenplasmaprobe ist.

16. Diagnostisches Testkit zum Durchführen eines diagnostischen Testverfahrens nach Anspruch 14 oder 15.

## Revendications

1. Molécule consistant en la séquence SÉQ ID : 1, lysine 5, qui est conjuguée au malondialdéhyde (MDA) et qui inhibe la captation des LDL par le récepteur à haute affinité des LDL.

2. Molécule consistant en la séquence SÉQ ID : 2, lysine 5, qui est conjuguée au MDA et qui inhibe la captation des LDL par le récepteur à haute affinité des LDL.

3. Molécule consistant en la séquence SÉQ ID : 3, lysine 7, qui est conjuguée au MDA et qui inhibe la captation des LDL par le récepteur à haute affinité des LDL.

4. Molécule selon l'une quelconque des revendications 1 à 3, qui inhibe la captation des LDL modifiées par conjugaison au MDA.

5. Molécule selon l'une quelconque des revendications 1 à 3, qui inhibe la captation des LDL modifiées par glycation.

6. Molécule selon l'une quelconque des revendications 1 à 3, qui inhibe la captation des LDL modifiées par conjugaison à des hydroxyalkénals.

7. Molécule selon la revendication 6, où la conjugaison est au 4-hydroxynonénal.

8. Molécule selon l'une quelconque des revendications précédentes, qui est utilisée dans une méthode de traitement ou de diagnostic du corps humain ou animal.

9. Utilisation d'une molécule selon l'une quelconque des revendications 1 à 7 dans la fabrication d'un médicament conçu pour inhiber la captation des LDL par le récepteur à haute affinité des LDL.

10. Méthode de fabrication d'un médicament conçu pour inhiber la captation des LDL par le récepteur à haute affinité des LDL, comprenant l'utilisation d'une molécule selon l'une quelconque des revendications 1 à 7.

11. Utilisation d'un anticorps qui se lie spécifiquement à une molécule selon l'une quelconque des revendications 1 à 7 dans la fabrication d'un médicament conçu pour inhiber la captation des LDL par le récepteur à haute affinité des LDL.

12. Méthode de fabrication d'un médicament conçu pour inhiber la captation des LDL ou de formes partiellement modifiées de celles-ci par le récepteur à haute affinité des LDL, comprenant l'utilisation d'un anticorps qui se lie spécifiquement à une molécule selon l'une quelconque des revendications 1 à 7.

13. Utilisation d'une molécule selon l'une quelconque des revendications 1 à 7 dans une méthode de diagnostic d'un anticorps spécifique des LDL oxydées causant la captation des LDL ou de formes partiellement modifiées de celles-ci par le récepteur à haute affinité des LDL.

14. Méthode de diagnostic d'un anticorps spécifique des LDL oxydées causant la captation des LDL ou de formes partiellement modifiées de celles-ci par le récepteur à haute affinité des LDL, qui comprend les étapes consistant à :
i) faire réagir une molécule selon l'une quelconque des revendications 1 à 7 avec un échantillon ;
ii) détecter une réaction de liaison anticorps-antigène ; et
iii) corréler la détection de la réaction de liaison anticorps-antigène à la présence d'un anticorps spécifique des LDL oxydées causant la captation des LDL ou de formes partiellement modifiées de celles-ci par le récepteur à haute affinité des LDL.

15. Méthode de diagnostic selon la revendication 14, où l'échantillon est un échantillon de plasma d'un patient.

16. Trousse diagnostique conçue pour effectuer une méthode de diagnostic selon la revendication 14 ou la revendication 15.


### SEQUENCE LISTING

(1) GENERAL INFORMATION:
   (i) APPLICANT:
      (A) NAME: University of Leicester
      (B) STREET: University Road
      (C) CITY: Leicester
      (E) COUNTRY: United Kingdom
      (F) POSTAL CODE (ZIP): LE1 7RH
   (ii) TITLE OF INVENTION: Oxidised LDL
   (iii) NUMBER OF SEQUENCES: 3
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (xi) SEQUENCE DESCRIPTION: SEQ ID NO: 1:
   Ala Leu Gln Tyr Lys Leu Glu Gly Thr Thr Arg
   1               5                   10
(2) INFORMATION FOR SEQ ID NO: 2:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (xi) SEQUENCE DESCRIPTION: SEQ ID NO: 2:
   Arg Leu Thr Arg Lys Arg Gly Leu Lys Leu Ala
   1               5                   10
(2) INFORMATION FOR SEQ ID NO: 3:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (xi) SEQUENCE DESCRIPTION: SEQ ID NO: 3:
   Ala Leu Ser Leu Ser Asn Lys Phe Val Glu Gly
   1               5                   10